# EUROPEAN PATENT SPECIFICATION

(11) **EP 1 360 282 B1**
(45) Date of publication and mention of the grant of the patent: **06.04.2005**
(21) Application number: 02726996.8
(22) Date of filing: 08.01.2002
(51) Int. Cl.: C12N 9/52, A61L 2/18, A61K 38/48, G01N 33/569, G01N 33/68, C07K 16/18, C07K 14/47, C11D 3/48

(54) **Method for inactivation of TSE**
Methode zur Inaktivierung von TSE
Méthode d'inactivation de la TSE

(30) Priority: 08.01.2001 GB 0100420; 26.02.2001 GB 0104696
(43) Date of publication of application: 12.11.2003
(60) Divisional application: 04025721.4
(73) Proprietor: Health Protection Agency, Salisbury, Wiltshire SP4 0JG (GB)
(72) Inventor: RAVEN, Neil D.H.,Health Protection Agency, Wiltshire SP4 0JG (GB)
(74) Representative: MacLean, Martin Robert
(86) International application number: PCT/GB2002/000052
(87) International publication number: WO 2002/053723

(56) References cited:
- WO-A-97/28192
- DE-A- 19 730 132
- US-A- 4 597 762
- US-A- 4 614 549
- US-A- 5 223 166
- US-A- 5 234 832
- US-A- 5 810 944
- KELLERSHOHN N ET AL: "SPECIES BARRIER IN PRION DISEASES: A KINETIC INTERPRETATION BASED ON THE CONFORMATIONAL ADAPTATION OF THE PRION PROTEIN" BIOCHEMICAL JOURNAL, PORTLAND PRESS, LONDON, GB, vol. PART 3, no. 334, 15 September 1998 (1998-09-15), pages 539-545, XP001070949 ISSN: 0264-6021
- BUSCHMANN A ET AL: "Cellular prion proteins of mammalian species display an intrinsic partial proteinase K resistance." BIOCHEMICAL AND BIOPHYSICAL RESEARCH COMMUNICATIONS, vol. 253, no. 3, 30 December 1998 (1998-12-30), pages 693-702, XP002214889 ISSN: 0006-291X
- WARWICKER JAMES: "Species barriers in a model for specific prion protein dimerisation." BIOCHEMICAL AND BIOPHYSICAL RESEARCH COMMUNICATIONS, vol. 232, no. 2, 1997, pages 508-512, XP002214890 ISSN: 0006-291X
- PERRETT SARAH ET AL: "Equilibrium folding properties of the yeast prion protein determinant Ure2." JOURNAL OF MOLECULAR BIOLOGY, vol. 290, no. 1, 2 July 1999 (1999-07-02), pages 331-345, XP002214891 ISSN: 0022-2836
- BIORESOURCE TECHNOLOGY, vol. 89, no. 1, pages 17-34,

## Description

The invention is in the field of methods and compositions for the sterilisation of materials and apparatus that may have been contaminated with infectious agents and for detection of those agents. In particular, the invention relates to methods for the inactivation and detection of transmissible spongiform encephalopathy (TSE) agents and provides compositions for degrading and detecting TSE located on or within infected materials.

Transmissible spongiform encephalopathies (TSEs) are a group of fatal neurological diseases that include Creutzfeld-Jacob disease (CJD) and Kuru in humans, bovine spongiform encephalopathy (BSE) in cattle and Scrapie in sheep. TSEs are characterised by the conversion of a normal host protein into a pathogenic protein within the brain tissue of an infected animal. The pathogenic form of protein is often referred to as a prion and is highly resistant to physical and chemical degradation. The prion is believed to be the transmissive agent through which the TSE disease is passed on between animals.

There has been considerable public alarm in recent years over the risks associated with consumption of meat products, and especially beef, potentially infected with BSE, the bovine form of TSE. Much of this concern is associated with the belief that the BSE prion when eaten by a human may in some cases cause the incurable human form of the disease, referred to as variant CJD (vCJD). Rigorous practices have been adopted in the agricultural and meat rendering industries to reduce the risk of cross contamination between BSE infected carcasses and meat that is destined for human consumption or other animal derived products such as tallow. However, BSE infected animals can still be unintentionally processed in abattoirs, especially if the animal is in the early stages of the disease and therefore undetected as an infected TSE host. There is also a considerable risk in disposal of known BSE infected material, particularly if the equipment used in the disposal operation is then reused in normal rendering practices without adequate sterilisation.

Sterilisation of instruments and equipment following potential exposure to TSE infected tissue is of primary importance. In particular, surgical equipment such as scalpels, forceps and endoscopes should be thoroughly sterilised before use on patients to avoid disease transmission.

It has been reported that the CJD infectious agent was accidentally transferred on surgical electrodes, inserted into the brain of a human patient with CJD, to two other previously uninfected patients (Bemouli et al (1977) The Lancet i: pp478-479). The electrodes concerned were sterilised with ethanol and formaldehyde vapour between each procedure, conditions previously thought to be sufficient to eliminate virtually all infectious agents, and yet the CJD infectious agent was able to withstand such harsh conditions and infect the recipient patients' brain tissue.

TSE transmission is typically observed in cases where infected material is transferred between animals or implanted into an animal. As described previously, incomplete or inadequate sterilisation of surgical instruments can lead to such transfer of infected material between patients. Even the most rigorous chemical cleaning and steam sterilisation procedures can fail to remove blood and tissue from surgical instruments, especially in the jaws or joints offorceps and clamps (Laurenson (1999) The Lancet, 20 Nov). Thus, the risk of unintentional TSE transfer can be unnecessarily high.

TSE agents, or prions, are known to be highly resistant to denaturation and degradation, more so than would normally be expected for a protein. Taylor (J. Hosp. Infect. (1999) 43 supplement, pp S69-76) reviews a number of methods for inactivating prion proteins.

Chemical methods for inactivating TSE prion proteins include treatment of infected material with sodium hydroxide or sodium hypochlorite solutions (Taylor et al. (1994) Arch. Virol. 139, pp313-326), although infectivity of the prion is shown to survive exposure to 2M sodium hydroxide for up to two hours.

Alternative methods for inactivating TSE prions include autoclaving, but again BSE and scrapie agent has been shown to survive treatment at 134-138°C for 18 minutes (Taylor et al. ibid). Thus, a combined chemical/heating approach has been proposed in which infected materials are exposed to 1M sodium hydroxide followed by autoclaving at 121°C for between 30 and 60 minutes (Taylor, J. Hosp. Infect. (1999) 43 supplement, pp S69-76). This combined method has shown that inactivation of TSE infectious agents can be achieved, albeit under very harsh conditions.

However, many materials, such as plastics, polymers and non-protein animal derivatives, cannot be exposed to such extreme conditions without themselves being destroyed. The chemical and physical processes described above are only really suitable for sterilising metal instruments and surgical tools that are not too large in size and which can fit inside a standard autoclave. More delicate instruments such as endoscopes cannot be exposed to extreme conditions of high temperature without the risk of permanent damage to their internal components.

Further, chemical processes typically involve the use of caustic and/or chaotropic agents which are hazardous to handle and dispose of. It would, therefore, be desirable to provide a method for inactivating TSE prions without the need to use large amounts of hazardous substances and which method could be scaled up for use on larger objects and areas as well as on smaller objects.

Taylor (Vet. J. (2000) 159 pp10-17) describes tests using proteolytic enzymes to deactivate prion proteins. Proteases such as trypsin have little effect in non-denaturing conditions (Taylor (2000) p. 14) but other proteases such as proteinase K may have an effect on TSE infectivity following prolonged digestion times. However, the majority of current TSE inactivation methods are aimed towards chemical and physical degradation procedures.

US Patent No. 5, 234, 832 describes the use of a proteolytic enzyme for cleaning and disinfecting medical instruments.

US Patent No. 4, 614, 549 describes the use of a proteolytic enzyme for cleaning and disinfecting contact lenses.

It is an object of the invention, therefore, to provide methods and means for effectively inactivating TSE infectious agents under conditions that can be readily applied to a variety of locations and situations. It is a further object of the invention to reduce the need for extreme conditions of very high temperature and harsh chemical denaturants in order to inactivate TSE agents located on or within TSE infected materials.

A first aspect of the invention provides a method for inactivating a TSE agent comprising exposing the TSE agent to a thermostable proteolytic enzyme at a temperature in the range 50-120°, wherein the enzyme is thermally stable and biologically active at a temperature in the range 50-120°.

The methods and compositions of the invention are suitable for the inactivation of TSE agent in apparatus and materials infected or suspected as being infected with TSE agent. In addition, the methods and compositions of the invention are suitably used in a prophylactic or precautionary mode, where definite knowledge of infection is uncertain. For example, the method of the invention can be easily incorporated into the standard sterilisation protocols used for preparation of surgical apparatus prior to use its use in surgical procedures.

The methods and compositions of the present invention are also suitably used for the inactivation of TSE agents in potentially contaminated clinical waste and culled animal material. At present, this waste material is incinerated at 1000°C, which requires specialised facilities and is expensive. It is an advantage of the present invention that a TSE inactivation procedure can occur at temperatures and in conditions which do not require highly specialised facilities and that the prospects of complete inactivation of the TSE agent are comparable to the more energy intensive and expensive incineration procedures.

The term transmissible spongiform encephalopathy (TSE) agent is intended to encompass all neurological diseases that are apparently transmitted via a pathogenic prion protein intermediate. Such TSEs typically include the human diseases Creutzfeld-Jacob disease (CJD), variant Creutzfeld-Jacob disease (vCJD), Kuru, fatal familial insomnia and Gerstmann-Straussler-Scheinker syndrome. Non-human TSEs include bovine spongiform encephalopathy (BSE), scraple, feline spongiform encephalopathy, chronic wasting disease, and transmissible mink encephalopathy. Given that vCJD is currently understood to be a human form of BSE, it is apparent that certain TSE agents are capable of crossing the species barrier and that novel TSEs from non-bovine sources could become evident in future.

The proteolytic enzyme of the invention is typically a protease but can be suitably any biological polymeric molecule capable of catalysing cleavage of a polypeptide chain.

It is a feature of the invention that the proteolytic enzyme is a thermostable enzyme, that is, that it demonstrates optimal biological activity at temperatures in excess of the normal mammalian body temperature of 37°C. In embodiments of the invention the enzyme is thermally stable and biologically active, and inactivation is carried out, at temperatures in the range of 50°C to 120°C; and more preferably where the temperature is between 55°C and 85°C. In a specific embodiment of the invention the temperature is about 60°C. In a further specific embodiment the temperature is about 80°C.

Thermostable proteolytic enzymes suitable for use in the methods and compositions of the invention are obtainable from a number of sources such as thermophilic bacteria and archaea. In one embodiment of the present invention the thermostable proteolytic enzyme is isolated from thermophilic bacteria, hyperthermophilic bacteria and archaea. Suitable organisms for extraction of proteolytic enzymes for use in the invention include *Thermotoga maritima; Thermotoga neopolitana; Thermotoga thermarum; Fervidobacterium islandicum; Fervidobacterium nodosum; Fervidobacterium pennivorans; Thermosipho africanus; Aeropyrum pemix; Thermus flavus; pyrococcus spp.; Sulfolobus solfataricus; Desulfurococcus; Bacillus thermoproteolyticus; Bacillus stearo-thermophilus; Bacillus sp. 11231; Bacillus sp. 11276; Bacillus sp. 11652; Bacillus sp. 12031; Thennus aquaticus; Thermus caldophilus; Thermus sp. 16132; Thermus sp. 15673;* and *Thermus sp. Rt41A*.

The aforementioned organisms are not the only sources of thermostable proteases. Indeed, some organisms that are not considered to be truly thermophilic can also express thermostable proteolytic enzymes. Such organisms are commonly termed thermodurable in that although they do not choose to live in conditions of high temperature, they can withstand high temperatures for limited periods. A number of Bacillus species fall in the category of thermodurability and are known to produce thermostable subtilisin-type proteases.

The pH at which the inactivation is performed can range from acid to alkaline, but is typically in the region of pH 8-13, preferably pH greater than 9 and more preferably around pH 12. Similarly, the thermostable protease is active in a pH range from acid to alkaline, but typically is optimally active in the region of pH 8-13, and preferably pH greater than 9 and more preferably around pH 12.

In an example of the invention in use, the proteolytic enzyme is extracted from a culture of the thermophilic bacteria or archaea. The culture is suitably maintained under the optimal conditions for the organism typically within a bioreactor. Thus, a continuous source of the organism can be maintained, allowing proteolytic enzyme to be obtained whenever needed.

Alternatively, in an example of the invention in use described in more detail below, the gene encoding a thermostable proteolytic enzyme is isolated from the source organism. *Bacillus thermoproteolyticus*. The gene is used to generate a recombinant expression construct, typically a plasmid, which is transformed into a host organism, *Escherichia coli*. The transformed *E. coli* is grown in a bioreactor and when at an appropriate cell density the expression of the plasmid construct is initiated and the proteolytic enzyme harvested using standard methods. The recombinant expression route allows for the production of the proteolytic enzyme product under less extreme conditions of temperature than would be required for the original source organism.

The recombinant route is further advantageous in that it allows for the genetic manipulation of recombinant thermostable protease genes in order to increase thermal stability or biological activity or for some other purpose. Thus, the activity of a thermostable proteolytic enzyme can be readily optimised for use in the methods and compositions of the invention.

A second aspect of the invention uses the method for sterilising apparatus, comprising the step of exposing the apparatus to a solution comprising a thermostable proteolytic enzyme.

The term "sterilising" is commonly understood to mean the procedure by which living organisms are removed from or killed in a substrate, such as a piece of equipment or a solution. In the present case the TSE agent, or prion, is not technically . considered to be a living organism, in the sense that a bacterium or virus is, because it does not apparently contain any genetic material. However, the transmission of the TSE pathogenic agent between animats does result in disease. Thus, the term "sterilising" as used herein is applied to the procedure by which both pathogenic agents (such as TSE agents) and living organisms are rendered non-infective or removed from or killed in a substrate.

In a preferred embodiment, the method of the invention comprises maintaining the sterilising solution at a temperature below 100°C, preferably at least 45°C and more preferably between 45°C and 85°C. The pH of the sterilising solution can range from acid to alkaline, but is typically in the region of pH 8-13, at least pH 9 and more preferably around pH 12. Similarly, the thermostable protease is active in a pH range from acid to alkaline, but typically is optimally active in the region of pH 8-13, at least pH9 and more preferably around pH 12.

In specific embodiments of the invention the sterilising solution is applied to the apparatus as a spray. The advantage of this mode of application is that, larger surface areas of apparatus, operating tables or even walls of rooms (for example in abattoirs) can be treated with the sterilising solution of the invention. Typically the solution will be heated to an optimal temperature, for example between 60°C to 80°C, before being sprayed onto the surface that is to be sterilised, that surface being optionally heated in advance.

Alternatively, the apparatus is immersed in the sterilising solution for a predetermined period of time. Again, the temperature of the solution is typically optimised prior to immersion of the contaminated apparatus. It is optional to include ultrasonication means in the immersion bath to enable ultrasonic cleaning to occur at the same time as treatment with the sterilising solution of the invention.

In a third aspect, the invention provides for a method of sterilising material, comprising exposing said material to a first solution comprising a thermostable proteolytic enzyme; and then exposing the apparatus to at least a second solution comprising a second thermostable proteolytic enzyme. In use, the material is typically apparatus, surgical or meat rendering equipment. or TSE infected biological waste.

By dividing the sterilisation method into at least two, and optionally more, successive steps the conditions in each step can be optimised to ensure maximum inactivation of any TSE agent present. Thus, the temperatures and/or pH of successive steps can be different. In specific embodiments of the invention the proteolytic enzymes in the first and second (and optionally more) solutions are the same, or are different.

In a fourth aspect the invention uses a composition for inactivating a TSE agent, comprising a thermostable proteolytic enzyme.

Typically, the composition of the invention further comprises a buffering agent. In a specific embodiment of the invention the buffering agent has a pKₐ of between 8 and 13. Alkaline buffers suitable for use in the method of the invention include 4-cyclohexylamino-1-butanesulfonic acid (CABS) which has a pKa of 10.7 at 25°C, and 3-cyclohexylamino-1-propanesulfonic acid (CAPS) which has a pKa of 10.4 at 25°C.

Alternatively, the composition comprises sufficient sodium hydroxide or other alkaline agent to adjust the pH of the composition to alkaline, preferably to at least pH 9 and more preferably around pH 12. Addition of 1M sodium hydroxide to the composition of the invention, using a pH probe calibrated using Universal standards, is generally sufficient to set the pH of the composition to around 12.

Further aspects of the invention provide for uses of the aforementioned compositions for the inactivation of TSE agents.

An advantage of the invention is its use in degrading TSE and similar agents, and it has been found in operation of particular embodiments of the invention that TSE-contaminated material has been successfully decontaminated using a combination of elevated temperature of around 50°C to 70°C and alkaline pH of around 9 to 12, with a thermostable, alkophilic proteolytic enzyme. Whilst it is on occasion possible to achieve some decontamination by extremely high temperature alone, it is of significant benefit to be able to inactivate TSE whilst avoiding extreme conditions, such as extremes of temperature, which lead to damage to the equipment being decontaminated.

The invention also provides dimer removal using protease degradation which is optionally enhanced by environmental conditions - high temperature, extremes of pH, and/or the use of detergents (e.g. SDS). As well as using single enzyme treatments, combinations of proteases and/or other enzymes can be used. For example, better degradation of the infective agent may be achieved by the addition of lipases, peptidases, glycosylases, nucleases and other enzymes.

To confirm dimer removal, a dimer cross-reactive antibody can be used in conjunction with a suitable detection system, one example being a sensitive in vitro detection system currently available from Invitrogen, referred to as Western Breeze, to confirm removal prior to further mouse bioassays.

Using the Western blot detection system, in an example below, the monomer is apparently completely removed by protease digestion. Under these conditions, however, samples which were subsequently used *in vivo* did not prevent infection and may even have enhanced its onset. There must, therefore, be another source of infection other than the monomer. Since the monomer can be removed (or at worst dramatically reduced in concentration) this indicates a primary role in infectivity for the dimer - either alone or in combination with the monomer.

The methods and compositions of specific embodiments of the inventions are described in more detail below and are illustrated by the accompanying drawings in which;
Fig. 1 shows the effect of temperature on a protease M digest of mouse brain homogenate (mbh);
Fig. 2 shows the effect of pH on a protease M digest of mbh;
Fig. 3 shows a *Bacillus thermoproteolyticus* Rokko digest of mbh;
Fig. 4 shows the effect of sodium dodecyl sulfate (SDS) on Rokko digest of mbh;
Fig. 5 shows and SDS-PAGE of mbh;
Fig. 6 shows and immunoblot of mbh;
Fig. 7 shows a protease G, R and C digest of mbh;
Fig. 8 shows an immunoblot of the digest in Fig. 7; and
Fig.s 9 to 12 show blots of BSE (301V)-infected mouse brain homogenate, to illustrate correlation of infectivity with prion dimer, and as further explained in the examples below.

### EXAMPLES

### VM Mouse Colony and Incubation with BSE (301V) agent

Studies on the inactivation of the TSE agent, BSE strain (301V), required the establishment of a mouse breeding colony for the generation of both uninfectious and infectious brain homogenate (mbh) and its subsequent titration and bioassay. The VM mouse strain selected for use in the study was obtained from Dr David Taylor (Institute of Animal Health, Edinburgh). Six pairs were introduced into a dedicated room within an animal facifity. Mice were screened for their health status and a breeding programme initiated.

BSE (301V) infectious mouse brain (IAH, Edinburgh) was prepared for inoculation by crude homogenisation followed by passage of the brains through increasingly fine gauge luer-locked needles (from 21G-27G) to and from a contained safety syringe into a closed septum-topped vial. This procedure was carried out in a validated safety cabinet within a containment level 3 (CL3) laboratory immediately prior to intracerebral inoculation of the VM mice. The anaesthetised (alphadolone/alphaxalone) mice were inoculated intracerebrally via 26 gauge needles with 20µl of the mouse brain homogenate preparation. Forty-nine out of fifty mice survived this procedure. These were retained to allow incubation of the agent and the generation of the required quantity of high-titre infectious material.

### Biomass Production

A wide variety of organisms were chosen for the production of biomass in order to provide as broad a selection of thermostable proteolytic enzymes as possible. Organisms selected ranged from those growing optimaliy at moderately thermophilic temperatures (50°C) through to extremely thermophitic temperatures (100°C) and included members of both the Archaea and the Bacteria. Thermophiles were also selected to cover a wide range of growth pH, encompassing pH optima from pH2.5 to pH11.5. The majority of organisms were grown in batch culture, however, where the growth requirements of the organism were particularly fastidious, continuous culture was used (Raven and Sharp (1997) Applied Microbial Physiology: A Practical Approach, Ch. 2, Eds. Stanbury and Rhodes, OUP pp.23-52). Depending on the biomass yield of the organism being grown, batch culture volumes of between 20L and 120L were employed to achieve the desired amount of cell paste. The continuous culture system utilised either a 2L or 5L working volume gas lift bioreactor constructed entirety of glass and PTFE. More prolific organisms such as *Bacillus* spp., and *Thermus* spp., were pre-screened to select those with high levels of protease activity prior to their culture on a larger scale. A quick and sensitive fluorometric protease assay utilising microtitre plates was. adopted for this purpose to permit high through-put screening (EnzChek™, Molecular Probes, Leiden, Netherlands).

Culture biomass was harvested by continuous centrifugation (Contifuge Stratos™, Kendro Laboratory Products, Bishop Stortford, UK) and stored at -80°C. Culture supernatants were concentrated with a 10KDa cut off tangential flow filter (Pall filtration, Portsmouth, UK). Proteins were precipitated with ammonium sulphate (90% saturation) and stored at -80°C.

### Protein Purification

A rapid protease screening and purification technique was required in order to process all of the crude protein preparations after the biomass production stage. A dye-ligand affinity chromatography system was used for this purpose (PIKSI M™, Affinity Chromatography Ltd., Isle of Man, UK). Initially, each crude ammonium sulphate precipitate was dissolved in buffer and passed through a desalting column. Each sample was then loaded onto the RIKSI M test kit, which contained 10 different affinity ligands. Fractions were then assayed for protease activity to determine the most suitable matrix for purification of the protease, either by positive binding of the target molecule and then elution, or by negative binding of contaminants. The purification was then scaled up using the same, affinity matrix in conjunction with an FPLC system (Amersham-Pharmacia Biotech, Amersham, UK). By combining this technique with the fluorometric protease assay, the rapid screening of many fractions could be undertaken.

The fluorometric protease assay utilises casein derivatives that are heavily labelled with green fluorescent BODIPY FL in which the conjugates' fluorescence is almost totally quenched. Protease catalysed hydrolysis releases the highly fluorescent label and the resultant fluorescence can be measured on a fluorometric microplate reader (Labsystems Fluoroscan II™). The increase in fluorescence is proportional to protease activity and was compared with that of a standard protease (Protease X, Sigma-Aldrich, Poole, UK).

### Protease Characterisation

A range of thermostable proteases were analysed (see Table 1). Direct characterisation of activity was carried out using the closest non-infectious analogue to BSE (301V) - infectious mouse brain homogenate available as substrate, i.e. normal VM mouse brain homogenate. Initial digests of total uninfected mouse brain homogenate (mbh) were performed over thirty minutes at 60°C and at pH7.0. The samples were then boiled under reducing conditions and analysed by SDS-PAGE on pre-cast NuPage 4 - 12% Bis-Tris gels (Novex™, San Diego, US). Gels were fixed using standard procedures and the proteins visualised using the Novex colloidal blue staining kit. The results showed both the levels of activity of the proteases against the mbh substrate under these conditions and also the level of purity of individual proteases. Protease concentrations used in the assays were based on total protein concentration, therefore, a wide range of activities was observed from limited to complete digestion.

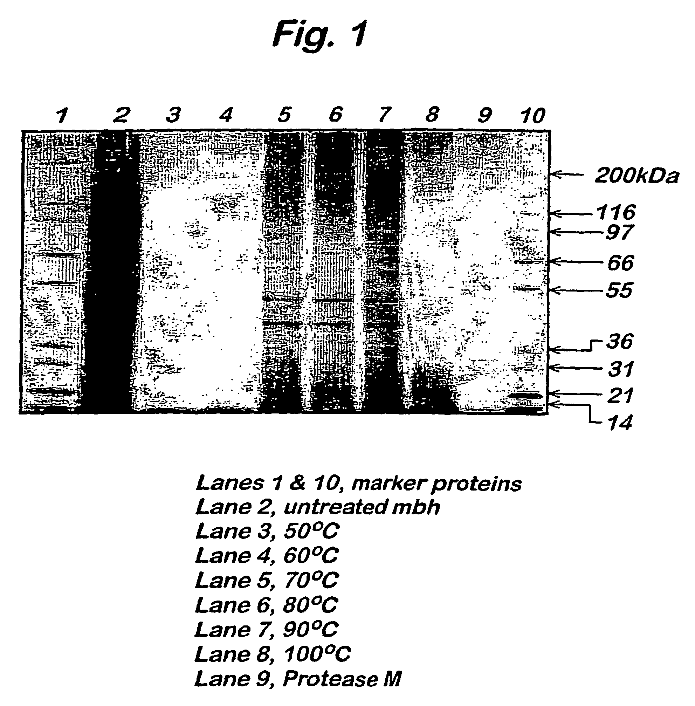

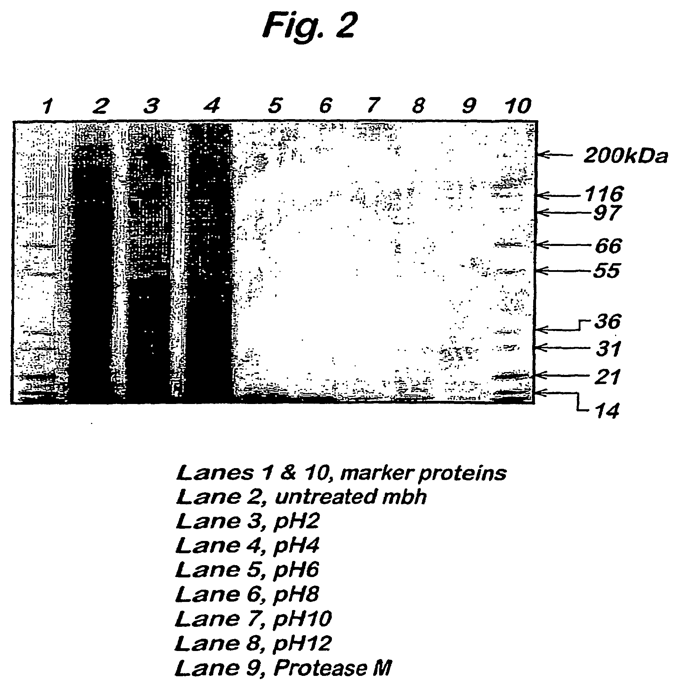

Activity profiles showing the full effect of pH and temperature on mbh digestion were then produced for each enzyme. Several enzymes showed extensive activity overthe complete range of conditions (pH 2 - 12 and 50 - 100°C). In general, however, complete digestion of mbh was only achieved over a narrower range indicative of the enzymes' pH and temperature optima. Figs. 1 and 2 show the activity profiles for a sample protease, *Bacillus* protease M. As can be seen the enzyme is moderately thermophilic and gives complete digestion at temperatures up to 70°C. Similarly the pH profile indicates a preference for neutral or alkaline conditions.

### Protease Testing

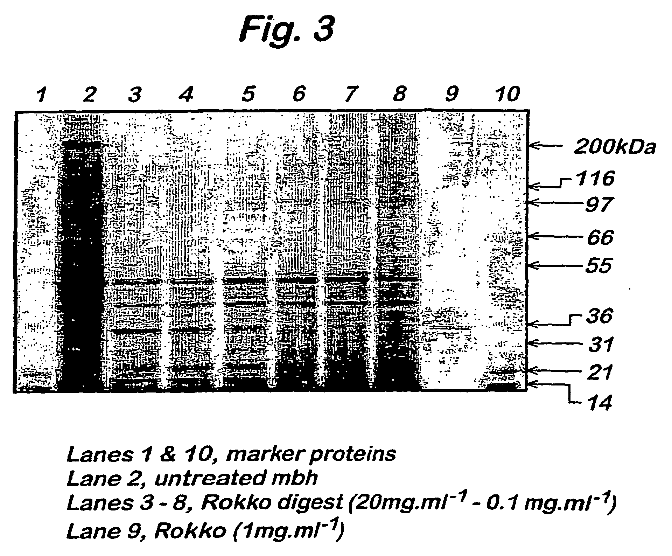

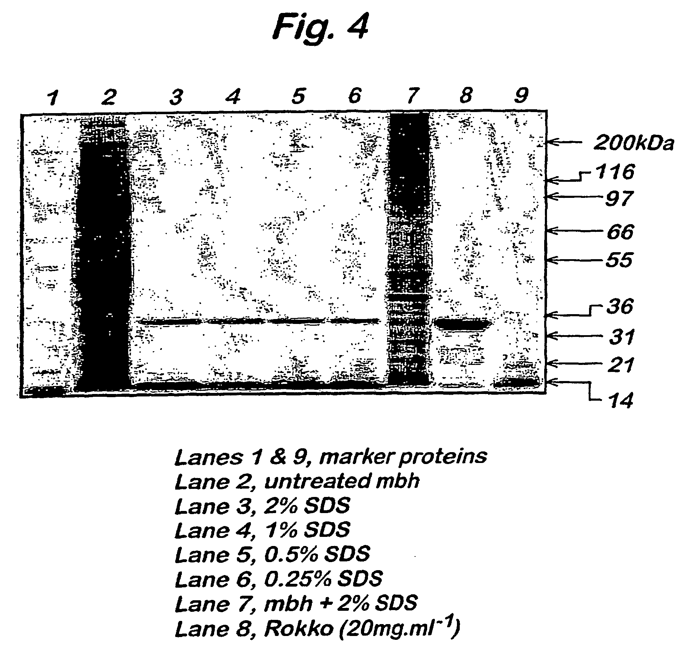

Several enzymes did not give complete digestion of mbh even when incubation times were increased to 24 hours. This may have simply been due to low protease activity, however, several highly purified and concentrated enzymes still only produced partial digests. In these reactions little enzyme-substrate interaction appeared to be occurring. it was considered that this could be due to non-proteinaceous material, e.g. lipids, surrounding the substrate and preventing interaction, however, pretreatment with lipases and other enzymes had no observable effect (data not shown). A second possibility considered was the effect of repulsive surface charges between proteases and the substrate. One enzyme in particular, purified from *Bacillus thermoproteolyticus* Rokko, consistently produced poor digestion of mbh even at high concentrations. This enzyme was known to remain active in the presence of high levels of detergents, therefore, digests were performed with the addition of SDS in an attempt to overcome this effect. Figures 3 and 4 show *B.thermoproteolyticus* Rokko digests of mbh in the absence and presence of SDS. Figure 3 clearly indicates that under standard conditions there is little difference in digestion even as the protease concentration is increased to 20mg.ml⁻¹. On addition of SDS (lanes 2-5), no protein bands, except the protease itself, are visible indicating a complete digest Tested proteases could, therefore, be divided simply into three categories: (i) those able to give total mbh digestion in the absence of detergent, (ii) those able to give total mbh digestion in the presence of SDS and (iii) those unable to digest the substrate fully. Proteases in categories. (i) and (ii) were selected for immediate further study, while those in category (iii) were either rejected or assumed to be required in greater quantity and/or higher purity.

### Western Blotting of Mouse Brain Homogenate

Mbh proteins were transferred onto nitrocellulose and blocked overnight in PBS-Tween (PBST) + 3% skimmed milk powder. The membrane was washed (x3 in PBST) and incubated for 1 hour with 6H4 anti-human recombinant PrP monoclonal antibody (Prionics, Zurich, Switzerland). After a second washing step, anti-mouse HRP-conjugate was added and the membrane incubated for 1 hour. Washing was repeated and the antibody reaction visualised by addition of TMB (Harlow and Lane (1988), Antibodies: A Laboratory Manual, Cold Spring Harbor Press).

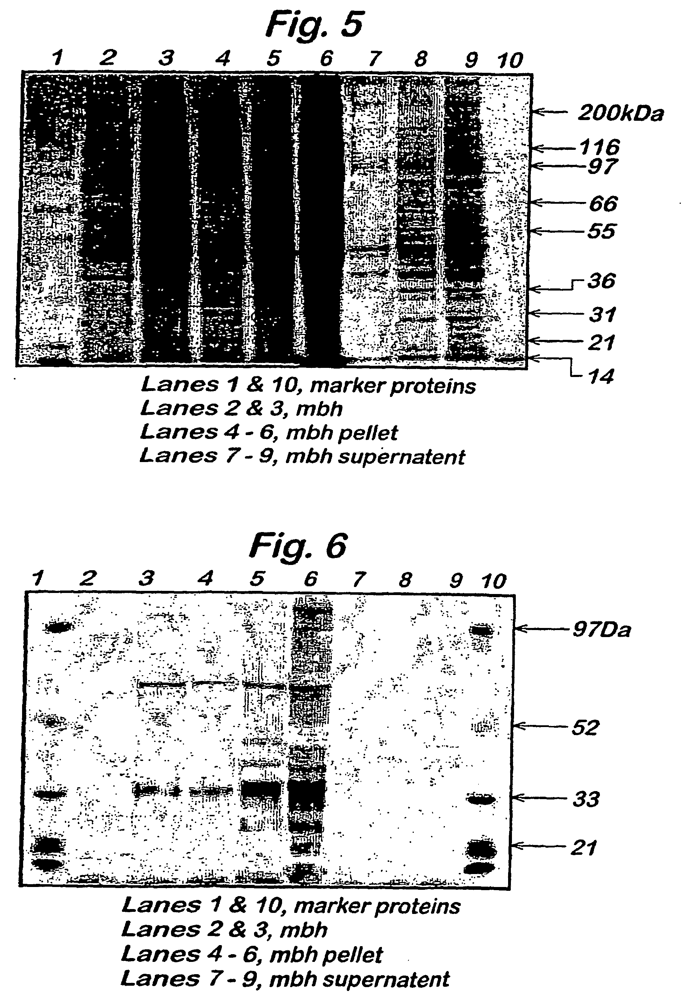

Figures 5 and 6 show an SDS-PAGE and an immunoblot of undigested mbh respectively. Although there is some non-specific background, dark bands indicating the presence of mouse prion can clearly be seen at the expected molecular weight (∼33-35kDa). Distinct bands are also visible at ∼66-70kDa, which may correspond to prion dimers previously reported (Safar *et al*. (1990) Proc. Natl. Acad. Sci. USA, 87:pp6373-6377).

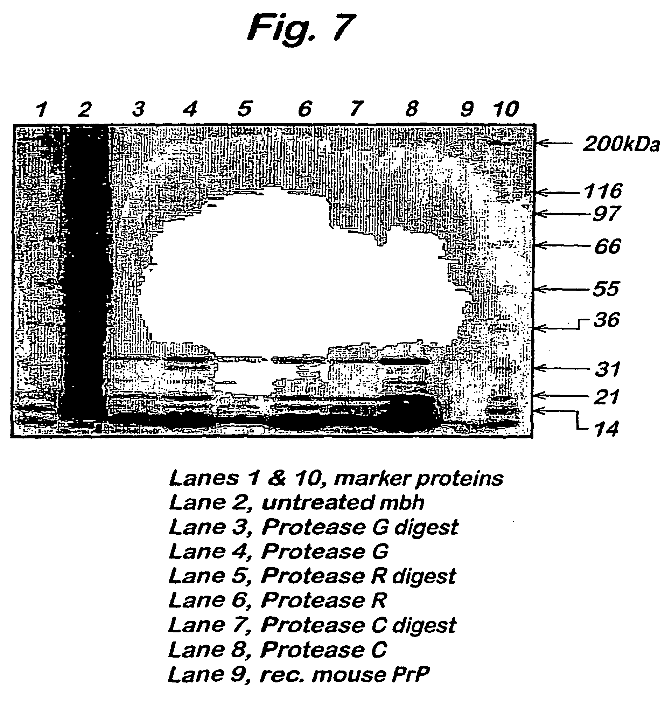

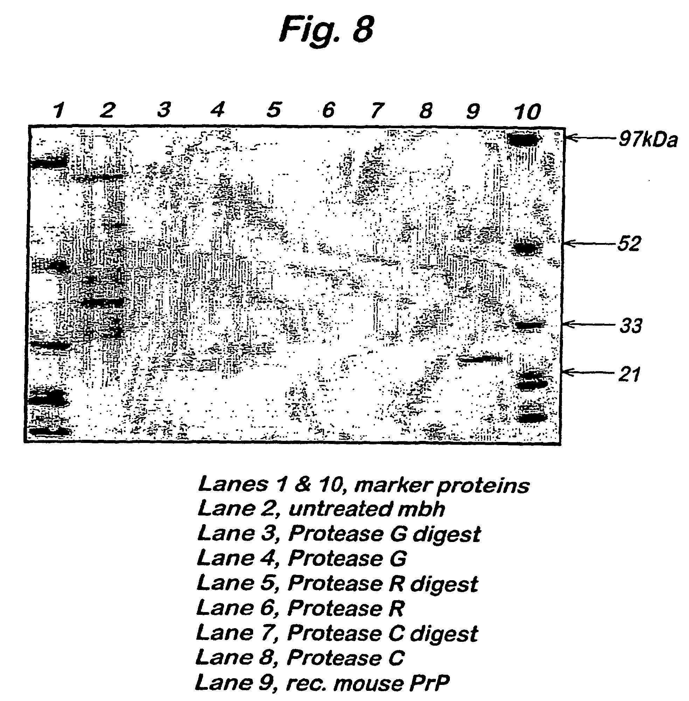

Western transfer and immunoblotting were used to confirm that no immunoreactive fragment of mouse PrP^{c} remained after digestion. Figure 7 shows mbh digested to completion with three closely related thermostable proteolytic enzymes (Proteases G, R and C), as assessed by SDS-PAGE. In the corresponding immunoblot (Figure 8) only 2 bands are visible, both with an apparent MW of ∼23kDa. The strong band in lane 9 corresponds to recombinant mouse PrP (+ve control), whilst the weak band in lane 4 appears to be due to a slight reaction with the heavily loaded enzyme preparation. In this case, complete loss of PrP^{c} immunoreactivity is still apparent since no band is observable in lane 3.

### Preparation and Titration of Infectious Mouse Brain Homogenate

Eighty days post challenge onward, mice were subject to daily clinical scoring to detect clinically affected mice as early as possible. A single mouse died of an unknown cause prior to this period. The remainder exhibited the expected disease progression and were sacrificed between 110 and 130 days post challenge. The brains were removed aseptically and stored frozen until required. Forty-eight BSE (301V) infectious VM mouse brains were homogenised in four volumes of PBS within a contained homogeniser then passed sequentially through increasingly fine gauge needles (21G to 26G) until free flowing. A sample with a further 2-fold dilution (1:9 mouse brain: PBS) was prepared for titration of infectivity. Over 800 x 0.1ml aliquots of BSE (301V) infectious mouse brain homogenate were prepared. These procedures were again carried out under rigorous class III containment, inducing the wearing of positive pressure respirators.

Groups of 25 eight week old VM mice received titration doses of the infectious mouse brain homogenate preparation at 10 fold dilutions from 10⁻¹ - 10⁻⁸. A further group of 25 mice were challenged with uninfectious mouse brain homogenate as a control. All mice were inoculated under anaesthetic using 26G x 3/8" (0.95cm) needles with plastic sleeve guards cut off 2mm below bevel in a Class 2 cabinet with the use of an injection guard. The mice were then left to incubate the BSE (301V) agent for extended periods some in excess of a year. The initial titre of the infectious mouse brain homogenate preparation was established retrospectively once all incubations (clinical monitoring at 80 days onwards) were complete.

### Dimer detection in digested mouse brain

BSE (301 V)-infected mouse brain homogenate was digested at neutral pH and 60°C for 30 minutes with protease. Total protein digests were run on SDS-PAGE and transferred by Western blotting to nitro-cellulose membranes. These were cut into strips and probed with CAMR anti-prion antibodies (produced in rabbits). A second generic antibody (goat anti-rabbit) was conjugated to horseradish peroxidase and used with detection by TMB colorimetric substrate.

At the time, the expected result was that the results were the same as the control blot (number 7). - using the anti-prion antibody mAb 6H4 (from Prionics, Switzerland). In this control blot, there is seen the typical three-banded pattem (glycosylation states) for protease-digested infectious-conformation prion protein (PrP^{Sc}).

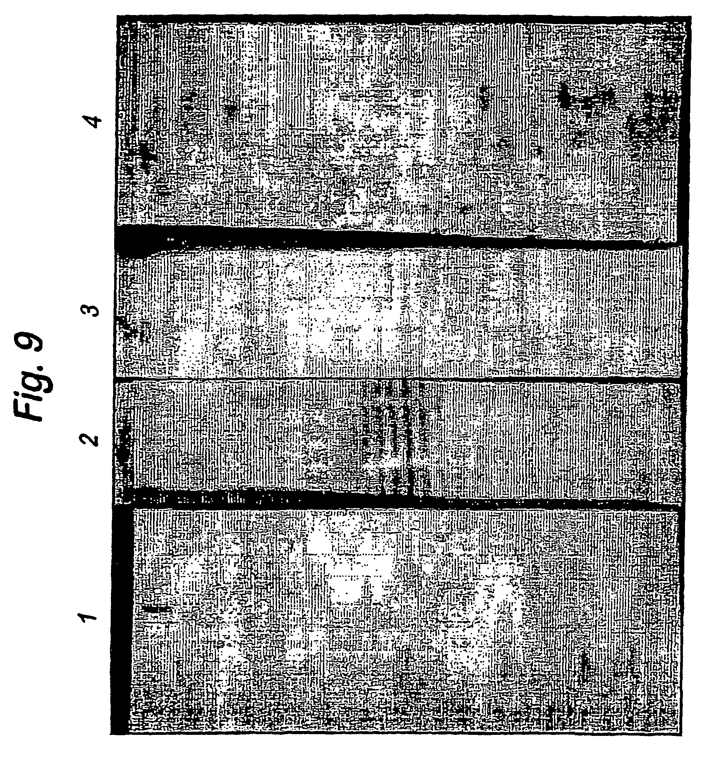

However, the blots in this example did not show this pattern. Blot 1 uses a polyclonal antibody raised against a PPD-conjugated peptide corresponding to an N-terminal region of the prion molecule. Nothing is seen in the lanes. This section of the protein is susceptible to proteolysis, so it is not surprising to see nothing in the lanes (2 & 3) - see figure 9, blot 1 on left hand side. Lane 1 is a molecular weight marker.

Blot 2 has a second antibody raised against a peptide sequence further into the prion molecule. This shows at least 9 bands of varying intensity, approximately equidistant, at a molecular, weight corresponding to a prion dimer with a range of glycosylation states - see blot 2 on figure 9.

Blot 3 antibody shows similar profile; blot 4 is also shown but its results are too poor quality to draw any conclusions - see blots 3 and 4 on figure 9.

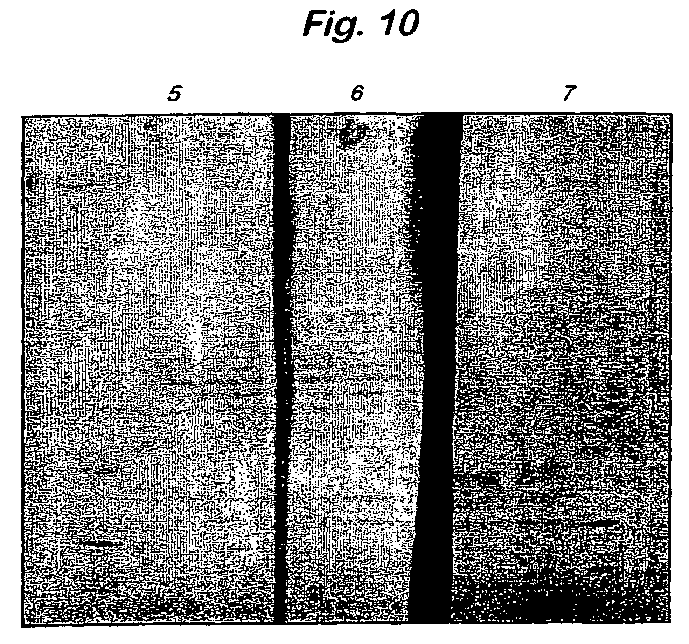

Blots 5 and 6, shown on figure 10 with the control blot 7, again show the multi-banded pattern

### Dimer detection in digested mouse brain

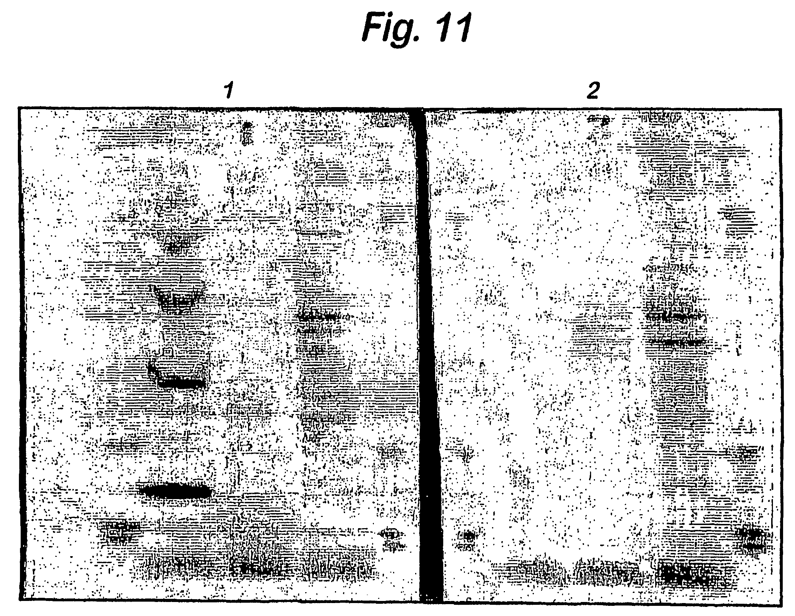

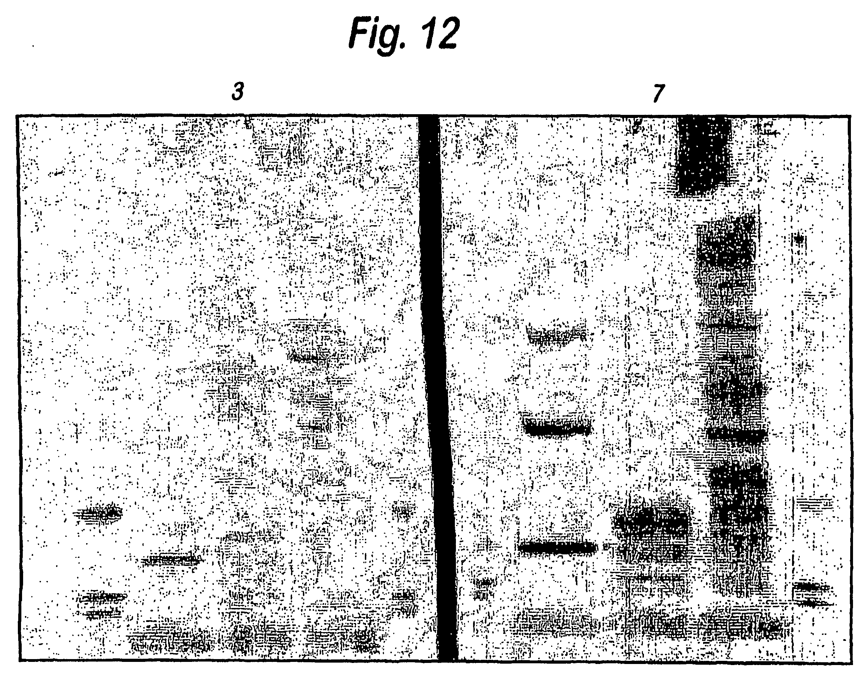

The above example was repeated, and the results shown in figures 11 and 12.

Blot 1 shows molecular weight markers in lanes 1 and 5. Lane 2 is recombinant murine PrP showing recombinant murine PrP oligomers. Lane 3 shows lack of antibody response to protease-digested infectious mouse brain homogenate. Lane 4 is the antibody response in the undigested control.

Blot 2 is as above but shows the previous banding pattern in the protease digested sample.

Blot 3 shows the antibody 3 response. Here there is some response to recombinant murine PrP (lane 2). Lane 3 shows not only the multiple (dimeric PrP) banding pattern, but also some monomeric PrP response.

Blot 7 is the 6H4 mAb antibody control. Here there is good detection of recombinant murine PrP oligomers (lane 2). Lane 3 shows the heavily diglycosylated form of limitedly protease-treated PrP^{Sc}, plus the more minor monoglycosylated and non-glycosylated forms typical of BSE (301V) strain. No 'dimer' detection is apparent.

### Preparation of antibodies including dimer preferential antibody

In the examples, we have used 6 polyclonal antibodies Of these, three detect the dimer alone and do not bind the monomer whereas one cross-reacts with both the monomer and the dimer.

The polyclonal sera were produced by immunisation of rabbits with synthesised prion mimetic peptides. These peptides were designed based on regions of high homology between human, mouse and bovine prion protein amino acid sequences.

The sequences producing the dimer-reactive antibodies were as follows:

The peptides were synthesised with a cysteine at both ends (see above) and with a cysteine at one end onty. This method was used in order to present both the linear form and a loop structure of the antigen on the surface of the carrier protein.

The peptides were synthesised commercially and coupled to the carrier protein PPD (purified protein derivative), derived from an attenuated strain of the bacterium *Mycobacterium bovis*, which is lyophilised and used to conjugate to the peptide via a linker.

Anti-prion polyclonal antibodies were produced as follows:

A sample of pre-immune sera (-1ml) was collected from each of a group of Dutch rabbits.

The rabbits were injected with reconstituted freeze-dried Bacillus Calmette-Guerin (BCG) vaccine for intradermal use. A dose of 0.1ml of reconstituted BCG vaccine was given in two sites in the scruff of the neck of the rabbit.

After 4 weeks, 0.6mg of each peptide-PPD conjugate was measured (0.3mg of each of the 1 cysteine and 2 cysteine versions) and dissolved in 1ml of sterile 0.9% saline.

An equal volume of incomplete Freunds adjuvant was added. and 0.75ml aliquots, of the resulting emulsion, were injected intra-muscularty into each hind limb and 0.25ml aliquots into two sites in the scruff of the neck per rabbit.

After 4 weeks a boost injection was given comprising of the peptide-PPD conjugates prepared as in step 3 and 4. The boost injections consist of four 0.25ml injections into the scruff of the neck of each rabbit.

7-14 days after the first boost injections, 4ml test bleeds were taken, the sera was assessed by ELISA for antibody titre.

A second boost injection was given 4-6 weeks after the first.

A third boost injection given 4-6 weeks later.

A 4ml test bleed was taken 6-8 weeks after the third boost injection and antibody titres determined by ELISA

A fourth boost injection given.

A 4ml test bleed was taken 7-14 days after the fourth boost injection and antibody titre determined by ELISA.

Terminal exsanguination was carried out and blood collected. The serum was separated by centrifugation and stored at -20°C.

Analysis of antibody titre was achieved using ELISA. The immunoassay plate was coated with the same peptides conjugated to a different carrier protein (KLH) in order to differentiate the response to the peptide from the response to the carrier protein.

Three of the antibodies produced by immunisation of the synthetic peptide sequences described bind preferentially to the dimer form of the molecule.

Analagous steps may also be used to prepare a monoclonal antibody. This could be achieved using a method such as described in Antibodies - A Laboratory Manual, Ed Harlow and David Lane, 1988 (Cold Spring Harbor Laboratory).

The invention thus provides detection and degradation of TSE infectivity.

**Table 1**

| **Organism** | **Domain** | **Growth** | **T opt** | **pH opt** |
|---|---|---|---|---|
| *Aeropyrum pernix* | Archaeon | Aerobe | 95°C | 7.0 |
| *Alicyclobacillus acidocaldarius* | Bacterium | Aerobe | 65°C | 3.5 |
| *Archaeoglobus fulgidus* | Archaeon | Anaerobe | 85°C | 6.5 |
| *Bacillus caldotenax BT1* | Bacterium | Aerobe | 65°C | 7.0 |
| *Bacillus pallidus* | Bacterium | Aerobe | 65°C | 9.0 |
| *Bacillus stearothermophilus L32-65* | Bacterium | Aerobe | 65°C | 7.0 |
| *Bacillus stearothermophilus LUDA T57* | Bacterium | Aerobe | 65°C | 7.0 |
| *Bacillus thermoproteolyticus Rokko* | Bacterium | Aerobe | 65°C | 7.0 |
| *Bacillus sp. 11231* | Bacterium | Aerobe | 65°C | 7.0 |
| *Bacillus sp. 11276* | Bacterium | Aerobe | 65°C | 7.0 |
| *Bacillus sp. 11652* | Bacterium | Aerobe | 65°C | 7.0 |
| *Bacillus sp. 12031* | Bacterium | Aerobe | 65°C | 7.0 |
| *Desulfurococcus sp*. | Archaeon | Anaerobe | 85°C | 6.5 |
| *Fervidobacterium pennivorans* | Bacterium | Anaerobe | 70°C | 8.5 |
| *Hyperthermus butylicus* | Archaeon | Anaerobe | 95°C | 6.5 |
| *Pyrococcus furiosus* | Archaeon | Anaerobe | 95°C | 7.5 |
| *Pyrococcus horikoshil* | Archaeon | Anaerobe | 95°C | 7.0 |
| *Sulfolobus acidocaldarius 98-3* | Archaeon | Aerobe | 75°C | 2.5 |
| *Sulfolobus hakonensis* | Archaeon | Aerobe | 75°C | 2.5 |
| *Sulfolobus solfataricus P1* | Archaeon | Aerobe | 75°C | 2.5 |
| *Sulfolobus solfataricus P2* | Archaeon | Aerobe | 75°C | 2.5 |
| *Thermobrachium celere* | Bacterium | Anaerobe | 65°C | 8.5 |
| *Thermococcus fumicolans* | Archaeon | Anaerobe | 85°C | 6.5 |
| *Thermus caldophilus GK24* | Bacterium | Aerobe | 70°C | 8.0 |
| *Thermus aquaticus YT1* | Bacterium | Aerobe | 70°C | 8.0 |
| *Thermus sp*. *16132* | Bacterium | Aerobe | 70°C | 8.0 |
| *Thermus sp*. *15673* | Bacterium | Aerobe | 70°C | 8.0 |
| *Thermus sp. Rt41A* | Bacterium | Aerobe | 70°C | 8.0 |

## Claims

1. A method for inactivating a transmissible spongiform encephalopathy (TSE) agent comprising exposing the TSE agent to a thermostable proteolytic enzyme at a temperature in the range 50-120°C, wherein the enzyme is thermally stable and biologically active at a temperature in the range 50-120°C.

2. A method according to claim 1, wherein the enzyme is thermally stable and biologically active at a temperature between 55°C and 85°C.

3. A method according to claim 2, wherein the enzyme is thermally stable and biologically active at a temperature of about 60°C.

4. A method according to claim 2, wherein the enzyme is thermally stable and biologically active at a temperature of about 80°C.

5. A method according to any of claims 1-4, comprising exposing the TSE agent to the enzyme at a temperature of between 55 and 85 °C.

6. A method according to claim 5, comprising exposing the TSE agent to the enzyme at a temperature of about 60 ° C.

7. A method according to claim 5, comprising exposing the TSE agent to the enzyme at a temperature of about 80 ° C.

8. A method according to any of claims 1-7, comprising exposing the TSE agent to the enzyme at alkaline pH.

9. A method according to claim 8, wherein the pH is between 8 and 13.

10. A method according to claim 8, wherein the pH is greater than 9.

11. A method according to claim 8, wherein the pH is about 12.

12. A method according to any previous claim wherein the TSE agent is a prion.

13. A method according to any previous claim, wherein the TSE is selected from the group consisting of Creutzfeld-Jacob disease; variant Creutzfeld-Jacob disease; Kuru; fatal familial insomnia; Gerstmann-Straussler-Scheinker syndrome; bovine spongiform encephalopathy; scrapie; feline spongiform encephalopathy; chronic wasting disease; and transmissible mink encephalopathy.

14. A method according to any previous claim wherein the proteolytic enzyme is obtained from a thermophilic organism.

15. A method according to claim 14, wherein the thermophilic organism is selected from the group consisting of archaea; hyperthermophilic bacteria and thermophilic bacteria.

16. A method according to claim 14 wherein the thermophilic organism is selected from the group consisting of *Thermotoga maritima; Thermotoga neopolitana; Thermotoga thermarum; Fervidobacterium islandicum; Fervidobacterium nodosum; Fervidobacterirsm pennivorans; Thermosipho africanus; Aeropyrum pernix; Thermus flavus; pyrococcus spp.: Sulfolobus solfataricus; Desulfurococcus; Bacillus thermoproteolyticus; Bacillus stearo-thermophilus; Bacillus sp. 11131; Bacillus sp. 11276; Bacillus sp. 11652; Bacillus sp. 12031; Thermus aquaticus; Thermus caldophilus; Thermus sp. 16132; Thermus sp. 15673; and Thermus sp. Rt41A*.

17. A method according to any previous claim for the inactivation of TSE agents in clinical waste.

18. A method according to any previous claim for the inactivation ofTSE agents in culled animal material.

19. A method according to any previous claim, comprising exposing the TSE agent to the enzyme at a temperature in the range 50-120°C, and wherein the pH is greater than 9.

20. A method of sterilising apparatus, comprising a method according to any of claims 1 to 19.

21. A method according to any of claims 1-20, wherein the thermostable proteolytic enzyme is in solution.

22. A method according to claim 21, wherein the temperature of the solution is maintained at below 100°C.

23. A method according to claim 21 or 22, wherein the solution is applied as a spray.

24. A method according to claim 21 or 22, wherein contaminated apparatus is immersed in the solution.

25. A method according to any of claims 1-24, wherein the TSE agent is exposed to a first solution comprising a proteolytic enzyme that is thermally stable and biologically active in the range 50-120°C; and then is exposed to a second solution comprising a proteolytic enzyme that is thermally stable and biologically active in the range 50-120°C.

26. A method according to claim 25, wherein the first and second proteolytic enzymes are the same.

27. A method according to claim 25, wherein the first proteolytic enzyme is different to the second proteolytic enzyme.

28. A method according to any of claims 25-27, wherein the pH of the first solution is different to the pH of the second solution.

29. A method according to any of claims 25-28, wherein the temperature of the first solution is different to the temperature of the second solution.

30. A method according to any of claims 1 to 29, further comprising confirming inactivation of TSE by confirming removal of prion dimer.

31. A method according to claim 30, comprising probing with an antibody specific to prion dimer.

32. Use of a composition comprising a thermostable proteolytic enzyme that is thermally stable and biologically active at a temperature in the range 50-120°C for sterilising material contaminated with a TSE agent.

33. Use of a thermostable proteolytic enzyme that is thermally stable and biologically active at a temperature in the range 50-120°C in the manufacture of a composition for inactivating a TSE agent.

## Patentansprüche

1. Verfahren zur Inaktivierung eines Mittels/Agens' der übertragbaren spongiformen Enzephalopathie (TSE), umfassend das Exponieren des TSE-Agens' an ein thermostabiles proteolytisches Enzym bei einer Temperatur im Bereich von 50-120°C, worin das Enzym thermisch stabil ist und bei einer Temperatur im Bereich von 50-120°C biologisch aktiv ist.

2. Verfahren nach Anspruch 1, worin das Enzym thermisch stabil und bei einer Temperatur zwischen 55°C und 85°C biologisch aktiv ist.

3. Verfahren nach Anspruch 2, worin das Enzym thermisch stabil und bei einer Temperatur von etwa 60°C biologisch aktiv ist.

4. Verfahren nach Anspruch 2, worin das Enzym thermisch stabil und bei einer Temperatur von etwa 80°C biologisch aktiv ist.

5. Verfahren nach einem der Ansprüche 1-4, umfassend das Exponieren des TSE-Agens' an das Enzym bei einer Temperatur von zwischen 55 und 85°C.

6. Verfahren nach Anspruch 5, umfassend das Exponieren des TSE-Agens' an das Enzym bei einer Temperatur von etwa 60°C.

7. Verfahren nach Anspruch 5, umfassend das Exponieren des TSE-Agens' an das Enzym bei einer Temperatur von etwa 80°C.

8. Verfahren nach einem der Ansprüche 1-7, umfassend das Exponieren des TSE-Agens' an das Enzym bei alkalischem pH-Wert.

9. Verfahren nach Anspruch 8, worin der pH-Wert zwischen 8 und 13 liegt.

10. Verfahren nach Anspruch 8, worin der pH-Wert größer als 9 ist.

11. Verfahren nach Anspruch 8, worin der pH-Wert etwa 12 beträgt.

12. Verfahren nach einem der vorstehenden Ansprüche, worin das TSE-Agens ein Prion ist.

13. Verfahren nach einem der vorstehenden Ansprüche, worin die TSE ausgewählt ist aus der Gruppe, bestehend aus der Creutzfeld-Jacob-Erkrankung, der abgewandelten Creutzfeld-Jacob-Erkrankung, Kuru, der tödlichen familiären Insomnie, dem Gerstmann-Straussler-Scheinker-Syndrom, der spongiformen Enzephalopathie des Rindes (Rinderwahn), Scrapie, der spongiformen Enzephalopathie der Katze; chronischer Auszehrung und der übertragbaren Nerzenzephalopathie.

14. Verfahren nach einem der vorstehenden Ansprüche, worin das proteolytische Enzym von einem thermophilen Organismus gehalten wird.

15. Verfahren nach Anspruch 14, worin der thermophile Organismus ausgewählt ist aus der Gruppe, bestehend aus Archaeen, hyperthermophilen Bakterien und thermophilen Bakterien.

16. Verfahren nach Anspruch 14, worin der thermophile Organismus ausgewählt ist aus der Gruppe, bestehend aus *Thermotoga maritima; Thermotoga neopolitana; Thermotoga thermarum; Fervidobacterium islandicum; Fervidobacterium nodosum; Fervidobacterium pennivorans; Thermosipho africanus; Aeropyrum pernix; Thermus flavus; pyrococcus spp.; Sulfolobus solfataricus; Desulfurococcus; Bacillus thermoproteolyticus; Bacillus stearo-thermophilus; Bacillus sp. 11231; Bacillus sp. 11276; Bacillus sp. 11652; Bacillus sp. 12031; Thermus aquaticus; Thermus caldophilus; Thermus sp. 16132; Thermus sp. 15673; und Thermus sp. Rt41A*.

17. Verfahren nach einem der vorstehenden Ansprüche zur Inaktivierung von TSE-Agenzien in klinischen Abfällen.

18. Verfahren nach einem der vorstehenden Ansprüche zur Inaktivierung von TSE-Agenzien in aussortiertem tierischen Material.

19. Verfahren nach einem der vorstehenden Ansprüche, umfassend das Exponieren des TSE-Agens' an das Enzym bei einer Temperatur im Bereich von 50-120°C, worin der pH-Wert größer als 9 ist.

20. Verfahren zum Sterilisieren von Vorrichtungen, umfassend ein Verfahren nach einem der Ansprüche 1 bis 19.

21. Verfahren nach einem der Ansprüche 1-20, worin sich das thermostabile proteolytische Enzym in Lösung befindet.

22. Verfahren nach Anspruch 21, worin die Temperatur der Lösung unterhalb von 100°C gehalten wird.

23. Verfahren nach Anspruch 21 oder 22, worin die Lösung als ein Spray aufgebracht wird.

24. Verfahren nach Anspruch 21 oder 22, worin die kontaminierte Vorrichtung in die Lösung eingetaucht wird.

25. Verfahren nach einem der Ansprüche 1-24, worin das TSE-Agens an eine erste Lösung, umfassend ein proteolytisches Enzym, das thermisch stabil und im Bereich von 50-120°C biologisch aktiv ist, exponiert wird und anschließend an eine zweite Lösung exponiert wird, umfassend ein proteolytisches Enzym, das thermischer stabil und in dem Bereich von 50-120°C biologisch aktiv ist.

26. Verfahren nach Anspruch 25, worin das erste und das zweite proteolytische Enzym gleich sind.

27. Verfahren nach Anspruch 25, worin sich das erste proteolytische Enzym von dem zweiten proteolytischen Enzym unterscheidet.

28. Verfahren nach einem der Ansprüche 25-27, worin der pH-Wert der ersten Lösung sich von dem pH-Wert der zweiten Lösung unterscheidet.

29. Verfahren nach einem der Ansprüche 25-28, worin die Temperatur der ersten Lösung sich von der Temperatur der zweiten Lösung unterscheidet.

30. Verfahren nach einem der Ansprüche 1-29, zusätzlich umfassend das Bestätigen der Inaktivierung von TSE durch Bestätigen der Entfernung des Prionen-Dimeren.

31. Verfahren nach Anspruch 30, umfassend das Sondieren mit einem für Prionen-Dimere spezifischen Antikörper.

32. Verwendung einer Zusammensetzung, umfassend ein thermostabiles proteolytisches Enzym, das thermisch stabil und bei einer Temperatur im Bereich von 50-120°C biologisch aktiv ist, zum Sterilisieren von mit einem TSE-Agens kontaminierten Material.

33. Verwendung eines thermisch stabilen proteolytischen Enzyms, das thermisch stabil und bei einer Temperatur im Bereich von 50-120°C biologisch aktiv ist, bei der Herstellung einer Zusammensetzung zum Inaktivieren eines TSE-Agens'.

## Revendications

1. Procédé pour inactiver un agent de l'encéphalopathie spongiforme transmissible (TSE) comprenant l'exposition de l'agent de la TSE à une enzyme protéolytique thermostable à une température dans la plage de 50 à 120°C, dans lequel l'enzyme est thermiquement stable et biologiquement active à une température dans la plage de 50 à 120°C.

2. Procédé selon la revendication 1, dans lequel l'enzyme est thermiquement stable et biologiquement active à une température entre 55°C et 85°C.

3. Procédé selon la revendication 2, dans lequel l'enzyme est thermiquement stable et biologiquement active à une température d'environ 60°C.

4. Procédé selon la revendication 2, dans lequel l'enzyme est thermiquement stable et biologiquement active à une température d'environ 80°C.

5. Procédé selon l'une quelconque des revendications 1 à 4, comprenant l'exposition de l'agent de la TSE à l'enzyme à une température entre 55°C et 85°C.

6. Procédé selon la revendication 5, comprenant l'exposition de l'agent de la TSE à l'enzyme à une température d'environ 60°C.

7. Procédé selon la revendication 5, comprenant l'exposition de l'agent de la TSE à l'enzyme à une température d'environ 80°C.

8. Procédé selon l'une quelconque des revendications 1 à 7, comprenant l'exposition de l'agent de la TSE à l'enzyme à un pH alcalin.

9. Procédé selon la revendication 8, dans lequel le pH est entre 8 et 13.

10. Procédé selon la revendication 8, dans lequel le pH est supérieur à 9.

11. Procédé selon la revendication 8, dans lequel le pH est environ 12.

12. Procédé selon l'une quelconque des revendications précédentes, dans lequel l'agent de la TSE est un prion.

13. Procédé selon l'une quelconque des revendications précédentes, dans lequel la TSE est choisie dans le groupe consistant en la maladie de Creutzfeld-Jacob ; la variante de la maladie de Creuzfeld-Jacob ; le kuru ; l'insomnie fatale familiale ; le syndrome de Gerstmann-Straussler-Scheinker ; l'encéphalopathie spongiforme bovine ; la tremblante du mouton ; l'encéphalopathie spongiforme féline ; la maladie du dépérissement chronique; et l'encéphalopathie transmissible du vison.

14. Procédé selon l'une quelconque des revendications précédentes, dans lequel l'enzyme protéolytique est obtenue à partir d'un organisme thermophile.

15. Procédé selon la revendication 14, dans lequel l'organisme thermophile est choisi dans le groupe consistant en les archéobactéries, les bactéries hyperthermophiles et les bactéries thermophiles.

16. Procédé selon la revendication 14, dans lequel l'organisme thermophile est choisi dans le groupe consistant en *Thermotoga maritima* ; *Thermotoga neopolitana* ; *Fervidobacterium islandicum* ; *Fervidobacterium nodosum* ; *Fervidobacterium pennivorans* ; *Thermosipho africanus* ; *Aeropyrum pernix* ; *Thermus flavus* ; *Pyrococcus spp* ; *Sulfolobus solfataricus* ; *Desulfurococcus* ; *Bacillus thermoproteolyticus* ; *Bacillus stearo-thermophilus* ; *Bacillus sp. 11231* ; *Bacillus sp. 11276* ; *Bacillus sp. 11652* ; *Bacillus sp. 12031* ; *Thermus aquaticus* ; *Thermus caldophilus* ; *Thermus sp. 16132* ; *Thermus sp. 15673* ; et *Thermus sp. Rt41A.*

17. Procédé selon l'une quelconque des revendications précédentes pour l'inactivation de la TSE dans les déchets cliniques.

18. Procédé selon l'une quelconque des revendications précédentes pour l'inactivation de la TSE dans les matières animales éliminées.

19. Procédé selon l'une quelconque des revendications précédentes, comprenant l'exposition de l'agent de la TSE à l'enzyme à une température dans la plage de 50 à 120°C, et dans lequel le pH est supérieur à 9.

20. Procédé de stérilisation d'un appareil, comprenant un procédé selon l'une quelconque des revendications 1 à 19.

21. Procédé selon l'une quelconque des revendications 1 à 20, dans lequel l'enzyme protéolytique thermostable est en solution.

22. Procédé selon la revendication 21, dans lequel la température de la solution est maintenue en dessous de 100°C.

23. Procédé selon la revendication 21 ou 22, dans lequel la solution est appliquée comme une vaporisation.

24. Procédé selon la revendication 21 ou 22, dans lequel l'appareil contaminé est immergé dans la solution.

25. Procédé selon l'une quelconque des revendications 1 à 24, dans lequel l'agent de la TSE est exposé à une première solution comprenant une enzyme protéolytique qui est thermiquement stable et biologiquement active dans la plage de 50 à 120°C ; et est ensuite exposé à une seconde solution comprenant une enzyme protéolytique qui est thermiquement stable et biologiquement active dans la plage de 50 à 120°C.

26. Procédé selon la revendication 25, dans lequel la première et la seconde enzymes protéolytiques sont la même.

27. Procédé selon la revendication 25, dans lequel la première enzyme protéolytique est différente de la seconde enzyme protéolytique.

28. Procédé selon l'une quelconque des revendications 25 à 27, dans lequel le pH de la première solution est différent du pH de la seconde solution.

29. Procédé selon l'une quelconque des revendications 25 à 28, dans lequel la température de la première solution est différente de la température de la seconde solution.

30. Procédé selon l'une quelconque des revendications 1 à 29, comprenant en outre la confirmation de l'inactivation de la TSE en confirmant la suppression du dimère du prion.

31. Procédé selon la revendication 30, comprenant le sondage avec un anticorps spécifique au dimère du prion.

32. Utilisation d'une composition comprenant une enzyme protéolytique qui est thermiquement stable et biologiquement active dans la plage de 50 à 120°C pour stériliser du matériel contaminé par un agent de la TSE.

33. Utilisation d'une enzyme protéolytique qui est thermiquement stable et biologiquement active dans la plage de 50 à 120°C dans la fabrication d'une composition pour inactiver un agent de la TSE.


### SEQUENCE LISTING

<110> Microbiological Research Authority Raven, Neil
<120> DEGRADATION AND DETECTION OF TSE INFECTIVITY
<130> GWS/22516
<150> GB 0104696.0
   <151> 2001-02-26
<150> GB 0100420.9
   <151> 2001-01-08
<160> 4
<170> PatentIn version 3.1
<210> 1
   <211> 11
   <212> PRT
   <213> synthetic
<400> 1
   Cys Gly Gly Trp Gly Gln Pro His Gly Gly Cys
   1               5                   10
<210> 2
   <211> 23
   <212> PRT
   <213> synthetic
<400> 2
   Cys Gly Gly Tyr Met Leu Gly Ser Ala Met Ser Arg Pro Ile Ile His
   1               5                   10                  15
   Phe Gly Asn Asp Tyr Glu Cys
               20
<210> 3
   <211> 25
   <212> PRT
   <213> synthetic
<400> 3.
   Cys Val Asn Ile Thr Ile Lys Gln His Thr Val Thr Thr Thr Thr Lys
   1               5                   10                  15
   Gly Glu Asn Phe Thr Glu Thr Asp Cys
               20                  25
<210> 4
   <211> 19
   <212> PRT
   <213> synthetic
<400> 4
   Cys Ile Thr Gln Tyr Gln Arg Glu Ser Gln Ala Tyr Tyr Gln Arg Gly
   1               5                   10                  15
   Ala Ser Cys